(19) Europäisches Patentamt / European Patent Office / Office européen des brevets

(11) **EP 3 168 650 B1**

(12) **EUROPEAN PATENT SPECIFICATION**

(45) Date of publication and mention of the grant of the patent:
**20.03.2019 Bulletin 2019/12**

(21) Application number: **15864666.1**

(22) Date of filing: **30.10.2015**

(51) Int Cl.:
*G01T 1/161* (2006.01)     *A61B 6/03* (2006.01)
*A61B 6/00* (2006.01)

(86) International application number:
**PCT/JP2015/080644**

(87) International publication number:
**WO 2016/088492 (09.06.2016 Gazette 2016/23)**

(54) **QUANTIFICATION OF NUCLEAR MEDICINE IMAGE DATA**

QUANTIFIZIERUNG VON NUKLEARMEDIZINISCHEN BILDDATEN

QUANTIFICATION DE DONNÉES D'IMAGES DE MÉDECINE NUCLÉAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2014 JP 2014244584**

(43) Date of publication of application:
**17.05.2017 Bulletin 2017/20**

(73) Proprietors:
• **Public University Corporation Yokohama City University**
  **Yokohama-shi, Kanagawa 236-0027 (JP)**
• **NIHON MEDI-PHYSICS CO., LTD**
  **Koto-ku**
  **Tokyo, 136-0075 (JP)**

(72) Inventors:
• **TATEISHI, Ukihide**
  **Yokohama-shi**
  **Kanagawa 236-0027 (JP)**
• **DAISAKI, Hiromitsu**
  **Tokyo 136-0075 (JP)**
• **NISHIDA, Kazumasa**
  **Tokyo 136-0075 (JP)**
• **HAMADA, Kazuo**
  **Tokyo 136-0075 (JP)**

(74) Representative: **V.O.**
**P.O. Box 87930**
**Carnegieplein 5**
**2508 DH Den Haag (NL)**

(56) References cited:
**JP-A- 2013 246 163     JP-A- 2014 174 654**
**JP-A- 2014 174 654**

• **DERLIN T ET AL: "Age-related differences in the activity of arterial mineral deposition and regional bone metabolism: a18F-sodium fluoride positron emission tomography study", OSTEOPOROSIS INTERNATIONAL, SPRINGER, GB, vol. 26, no. 1, 16 August 2014 (2014-08-16), pages 199-207, XP035422192, ISSN: 0937-941X, DOI: 10.1007/S00198-014-2839-6 [retrieved on 2014-08-16]**
• **John W. Keyes: "SUV: Standard Uptake or Silly Useless Value?", The Journal of Nuclear Medicine, 30 October 1995 (1995-10-30), pages 1836-1839, XP055449621, Retrieved from the Internet: URL:http://jnm.snmjournals.org/content/36/10/1836.full.pdf#page=1&view=FitH [retrieved on 2018-02-08]**
• **A. Thie: "Understanding the Standardized Uptake Value, Its Methods, and Implications for Usage", THE JOURNAL OF NUCLEAR MEDICINE, 30 September 2004 (2004-09-30), pages 1431-1434, XP055449623, Retrieved from the Internet: URL:http://jnm.snmjournals.org/content/45/9/1431.full.pdf#page=1&view=FitH [retrieved on 2018-02-08]**

**Description**

FIELD

The present invention relates to processing of nuclear medicine image data. More particularly, the present invention relates to providing a novel technique for objectively evaluating results of nuclear medicine examinations.

BACKGROUND

The principle of imaging technology based on nuclear medicine techniques, such as positron emission tomography (PET), single-photon emission computed tomography (SPECT), and scintigraphy, is to perform imaging by administering an agent (radiopharmaceutical) labeled with radioactive material into a subject's body and detecting radiation emitted from within the body. A tissue having an abnormality indicates different metabolism of the radiopharmaceutical from that in normal tissues. Thus, functional abnormality of tissue can be imaged by selectively using a radiopharmaceutical having desired metabolic characteristics.

PET, SPECT, and other similar techniques provide primary images that are visualized values of radiation count values. In such images, sites having high-concentration of radiopharmaceutical are illuminated. In other words, the pixels corresponding to the sites have high pixel values. Radioactivity count values, however, are subject to various factors, and thus higher pixel values of specific pixels or in a region of interest do not always indicate abnormality in the corresponding tissue. Thus, efforts have been made to normalize each pixel value according to some rules so as to enable objective evaluation of pixel values. The most famous one of such normalizing values is the standardized uptake value (SUV). The SUV is obtained as follows:

[Formula 1]

$$SUV = \{\text{Amount of attenuation-corrected radioactivity in region of interest (kBq)} \div \text{Volume of region of interest (ml)}\} / \{\text{Administered radioactivity dose (MBq)} \div \text{Body weight of subject (kg)}\}$$

Thus, the SUV is a value obtained by normalizing the radioactivity concentration in a region of interest with the administered radioactivity dose per kg body weight. The SUV can be an indicator that reflects the uptake of radioactivity or a radiopharmaceutical.

With regard to tissues working in a similar manner, the SUV has been expected to take similar values independent of different subjects. In other words, the SUV is expected to have such characteristics that it takes, for example, similar values for liver tissues having no abnormality regardless of different subjects, and, for example, similar values for lung tissues having no abnormality regardless of different subjects. If this is true, comparison is possible among a plurality of measurement results from different subjects, or from the same subject at different times. (In the technical field pertaining to the present application, converting a measured value to a comparable value is referred to as "quantification".) A modified SUV has recently been used, which is obtained by using body weight excluding body fat (referred to as lean body weight) instead of using body weight as it is.

LIST OF PRIOR ART DOCUMENTS

NON PATENT LITERATURE

Non Patent Literature 1: Yoshifumi Sugawara, Kenneth R. Zasadny et al., "Reevaluation of the Standardized Uptake Value for FDG: Variations with Body Weight and Methods for Correction", November 1999 Radiology, 213, 521-525.

SUMMARY

However, the inventors of the present application are skeptical about the use of the SUV when radiopharmaceuticals used are bone disease diagnostic agents. Bone disease diagnostic agents, which are radiopharmaceuticals used for detecting, for example, bone metastases of cancer, are distributed to bones. The SUV is a value normalized with body weight and is not a value normalized with information on bones.

The inventors of the present application have performed an experiment on the following group of subjects to verify the validity of the SUV:

- Number of subjects: 15 (8 males and 7 females)
- Age: 57 to 79 (67 on average)
- Body weight: 39 to 69 kg (53.3 kg on average)

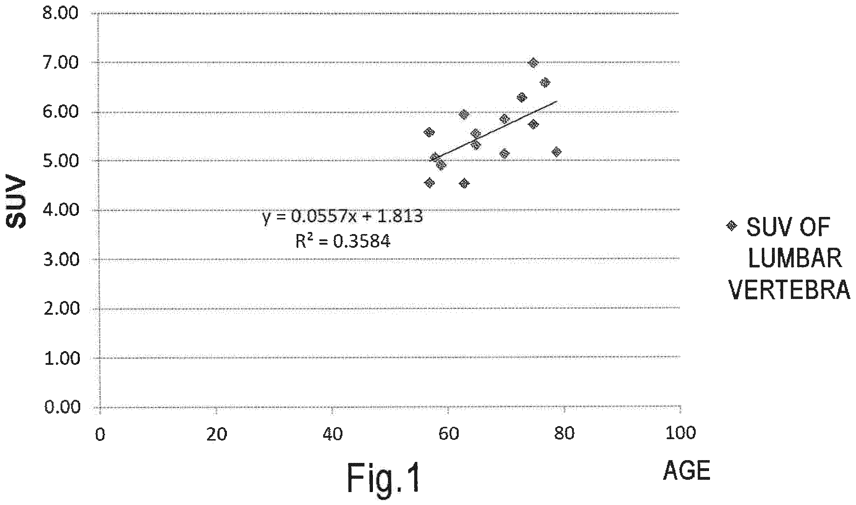

The inventors of the present application performed PET measurement on the subjects by administering $^{18}$F-NaF as a bone disease diagnostic agent, calculated SUVs by setting a region of interest to the lumbar vertebrae in the obtained image data, and plotted the results on a graph having the age on the abscissa axis. The administered radioactivity doses were 133.7 to 217.8 MBq, the average of which was 176.1 MBq. The results are indicated in FIG. 1.

As the graph in FIG. 1 clearly illustrates, age dependency is recognized from the results. For reference, fitting the results to a straight line establishes the following:

$$y = 0.0557x + 1.813.$$

Here, $R^2 = 0.3584$ is established. Testing a null hypothesis with a level of 5%, which states that the slope of the regression line is not significant, resulted in $P = 0.018$, and hence the significance of the slope is recognized.

In other words, the experiment performed by the inventors of the present application has revealed that the SUV has age dependency with respect to the bone disease diagnostic agent. This suggests that the SUV may possibly be inappropriate as an indicator that reflects the uptake of bone disease diagnostic agents. If this is the case, an urgent task is to establish a novel technique of quantifying nuclear medicine data for bone disease diagnostic agents.

The inventors of the present application have focused on bone mineral content (BMC) and bone mineral density (BMD) as indicators related to bones in which bone disease diagnostic agents are distributed. The inventors consider that the stability of quantitative assessment on bone disease diagnostic agents can be improved by using BMC or BMD for normalization of nuclear medicine images instead of using body weight, which has been used for the conventional SUV.

On the basis of the consideration, the inventors of the present application provide the SUVbone defined by the following formula 3 or 4, instead of the conventional SUV, as an indicator reflecting the uptake of bone disease diagnostic agents, for example.

```
[Formula 2]

SUVbone = {Amount of attenuation-corrected radioactivity in
region of interest (kBq) ÷ Volume of region of interest
(ml)} / {Administered radioactivity dose (kBq) ÷ Bone
mineral content (g)}
```

```
[Formula 3]

SUVbone = {Amount of attenuation-corrected radioactivity in
region of interest (kBq) ÷ Volume of region of interest
(ml)} / {Administered radioactivity dose (kBq) ÷ Bone
mineral density (g/m²)}
```

In formulas 2 and 3, the "region of interest" may be a region corresponding to one pixel, or a region including a plurality of pixels.

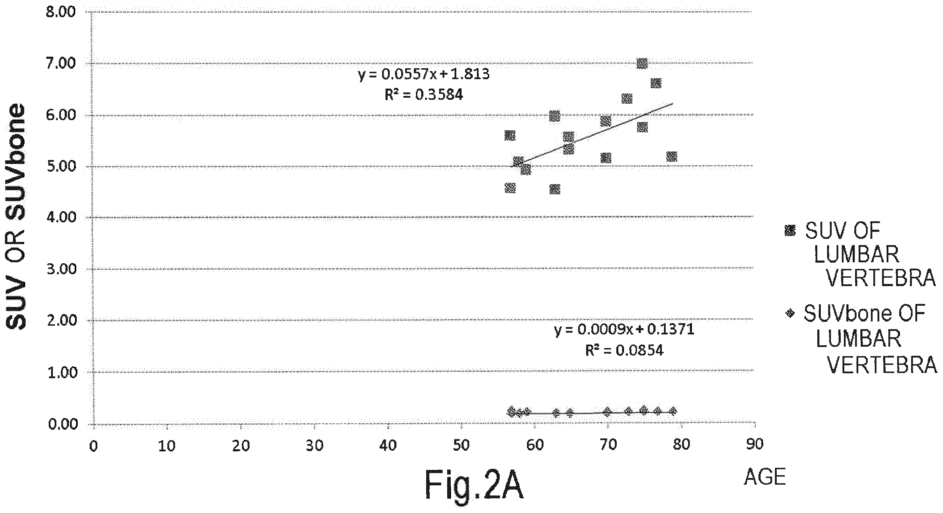

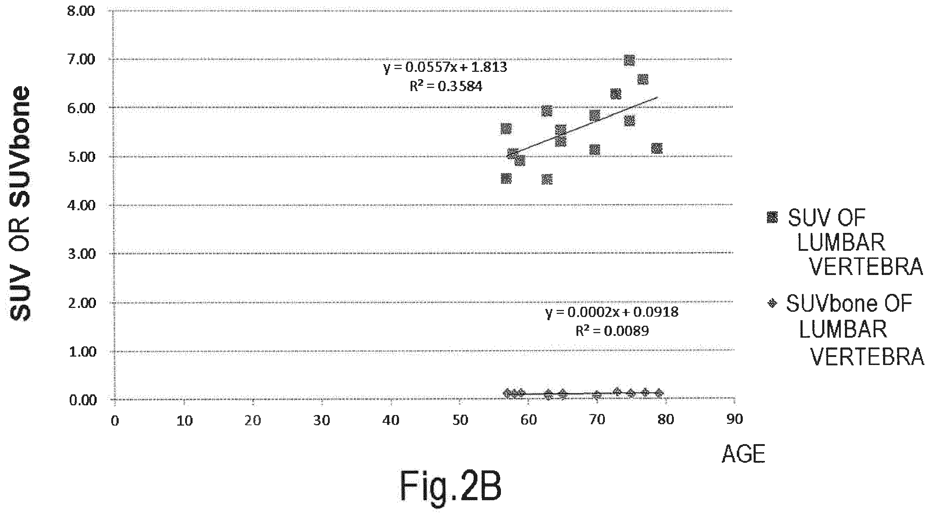

FIG. 2 illustrates the data indicating an advantage of the SUVbone.

FIG. 2A illustrates a graph of the same experimental data as that of FIG. 1. The graph has been created by setting the same region of interest as that provided the results in FIG. 1 (that is, setting the region of interest to the lumbar vertebra), calculating the SUVbones defined by formula 2, and plotting the results on the graph having the age on the abscissa axis. For comparison, the same conventional SUVs as that illustrated in FIG. 1 are plotted on the graph. As is clear from the graph, the SUVbone is extremely low in age dependency, and indicates a substantially constant value regardless of age. For reference, fitting the SUVbones to a straight line establishes the following:

$$y = 0.0009x + 0.1371.$$

Here, $R^2 = 0.0854$ is established. That is, effective age dependency is not recognized. In order to confirm its statistical significance, the null hypothesis that the slope of the regression line is not significant was tested with a level of 5%. This test resulted in $P = 0.29$, and hence the slope of the regression line is not recognized statistically as well.

This indicates that the invention of the present application that uses BMC to normalize nuclear medicine images is extremely advantageous in quantifying the uptake of the radiopharmaceuticals characterized by concentration in bones.

The following describes the data indicating an advantage of the SUVbone defined by formula 3. FIG. 2B illustrates a graph of the same experimental data as that of FIG. 1. The graph has been created by setting the same region of interest as that provided the results in FIG. 1 (that is, setting the region of interest to the lumbar vertebra), calculating the SUVbones defined by formula 3, and plotting the results on the graph having the age on the abscissa axis. In the same manner as FIG. 2A, the same conventional SUVs as that illustrated in FIG. 1 are plotted on the graph for comparison. As is clear from the graph, the SUVbone defined by formula 3 is extremely low in age dependency, and indicates a substantially constant value regardless of age. For reference, fitting the SUVbones to a straight line establishes the following:

$$y = 0.0002x + 0.0918.$$

Here, $R^2 = 0.0089$ is established. That is, effective age dependency is not recognized. In order to confirm its statistical significance, the null hypothesis that the slope of the regression line is not significant was tested with a level of 5%. This test resulted in $P = 0.73$, and hence the slope of the regression line is not recognized statistically as well. That is, the slope of the regression line is statistically zero, and the age dependency of the SUVbone has been proved to be not present statistically.

These results indicate that the invention of the present application that uses BMD to normalize nuclear medicine images is extremely advantageous in quantifying the uptake of the radiopharmaceuticals characterized by concentration in bones.

Thus, embodiments of the invention of the present application are generally characterized in that information acquired from nuclear medicine image data is normalized with bone mineral content (BMC) or bone mineral density (BMD).

In some embodiments, BMC or BMD may be estimated from sex, age, height, or weight of a subject.

An example of preferred embodiments of the invention of the present application is a system. The system includes:

first means for loading nuclear medicine image data;
second means for loading sex information on a subject of the nuclear medicine image data;
third means for loading age information on the subject;
fourth means for loading height information on the subject;
fifth means for loading weight information on the subject;
sixth means for loading information on a radioactivity dose administered to the subject;
seventh means for acquiring certain information from the nuclear medicine image data;
eighth means for calculating bone mineral content or bone mineral density based on at least one of the sex information acquired by the second means, the age information acquired by the third means, the height information acquired by the fourth means, or the weight information acquired by the fifth means;
ninth means for normalizing the certain information acquired by the seventh means based on the administered radioactivity dose information acquired by the sixth means, and the bone mineral content or the bone mineral density acquired by the eighth means; and
tenth means for outputting one or more normalized values acquired by the ninth means.

An example of preferred embodiments of the invention of the present application is a computer program. The computer program includes a program instruction that causes, when executed by processing means of a system, the system to perform:

loading nuclear medicine image data;
loading sex information on a subject of the nuclear medicine image data;
loading age information on the subject;
loading height information on the subject;
loading weight information on the subject;
loading information on a radioactivity dose administered to the subject;
acquiring certain information from the nuclear medicine image data;
calculating bone mineral content or bone mineral density based on at least one of the sex information, the age information, the height information, or the weight information;
normalizing the certain information based on the information on the administered radioactivity dose, and the bone mineral content or the bone mineral density; and
outputting the normalized information.

An example of preferred embodiments of the invention of the present application is a system. The system includes:

first means for loading nuclear medicine image data;
second means for loading information on a radioactivity dose administered to a subject of the nuclear medicine image data;
third means for loading information on bone mineral content or bone mineral density of the subject;
fourth means for acquiring certain information from the nuclear medicine image data;
fifth means for normalizing the certain information acquired by the fourth means based on the administered radioactivity dose information acquired by the second means, and the bone mineral content or the bone mineral density acquired by the third means; and
sixth means for outputting one or more normalized values acquired by the fifth means.

An example of preferred embodiments of the invention of the present application is a computer program. The computer program includes a program instruction that causes, when executed by processing means of a system, the system to perform:

loading nuclear medicine image data;
loading information on a radioactivity dose administered to a subject of the nuclear medicine image data;
loading information on bone mineral content or bone mineral density of the subject;
acquiring certain information from the nuclear medicine image data;
normalizing the certain information based on the information on the administered radioactivity dose, and the bone mineral content or the bone mineral density; and
outputting the normalized information.

Several preferred embodiments of the present invention are specified in the claims included in the attached claims. However, the embodiments specified in the claims do not necessarily include all the novel technical ideas disclosed in the present description and the drawings. The applicants claim to possess the right to have a patent granted on all the novel technical ideas disclosed in the present description and the drawings regardless of whether the novel technical ideas are claimed in the current claims.

BRIEF DESCRIPTION OF DRAWINGS

FIG. 1 is a graph illustrating that the SUV has age dependency in diagnosis of bone metastases using a nuclear medicine technique.

FIG. 2A is a graph illustrating that the SUVbone (using BMC) developed by the inventors of the present application indicates no age dependency.

FIG. 2B is a graph illustrating that the SUVbone (using BMD) developed by the inventors of the present application indicates no age dependency.

FIG. 3 is an explanatory diagram of a system configuration that can embody the present invention.

FIG. 4 is an explanatory diagram of exemplary processing according to the present invention.

DESCRIPTION OF EMBODIMENTS

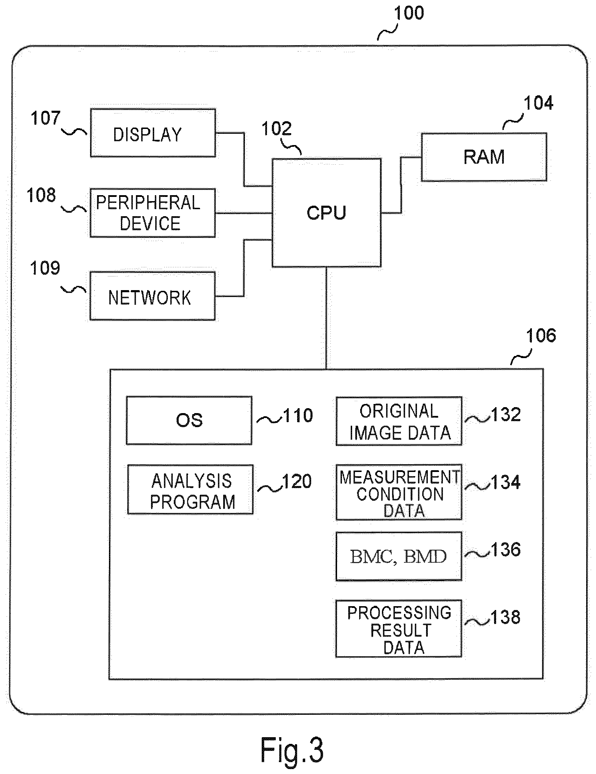

FIG. 3 is a diagram illustrating a main configuration of an apparatus or a system 100, which is exemplary hardware capable of executing various processes disclosed in the present description. As illustrated in FIG. 3, the system 100 is similar to a general-purpose computer in terms of hardware, and may include a central processing unit (CPU) 102, a main memory 104, an auxiliary storage unit 106, a display interface 107, a peripheral device interface 108, and a network interface 109, for example. Similarly to a general-purpose computer, a highspeed random access memory (RAM) may be used as the main memory 104, and an inexpensive, large-capacity hard disk or a solid state disk (SSD) may be used as the auxiliary storage unit 106. To the system 100, a display for displaying information may be connected through the display interface 107. Also to the system 100, a user interface such as a keyboard, a mouse, or a touch panel may be connected through the peripheral device interface 108. The network interface 109 may be used to connect the system 100 to another computer or the Internet via a network.

The auxiliary storage unit 106 may store therein, for example, an operating system (OS) 110 and an analysis program 120 for providing characteristic processes disclosed in the present description. The most basic functions of the system 100 are provided when the CPU 102 executes the OS 110.

In addition, novel processes disclosed in the present description are provided when the CPU 102 executes the analysis program 120. A program instruction group constructing the analysis program 120 may be programmed in any of existing computer program languages such as C++ and JAVA (registered trademark). The program instruction group may be converted to an executable format by a preferred compiler and stored in the auxiliary storage unit 106.

The auxiliary storage unit 106 may store therein, for example, nuclear medicine image data 132 subject to an analysis by the analysis program 120, measurement condition data 134 containing various measurement conditions of the nuclear medicine image data 132, data 136 containing information calculated during processes, and data 138 containing results of the processes.

Other than the components illustrated in FIG. 3, the system 100 may include the same units as those of a general computer system, such as a power source and a cooling unit. Various embodiments of a computer system employing various techniques have been known, such as distributed, redundant, or virtualized storage units, use of multiple CPUs, CPU virtualization, use of a processor suitable for a specific process such as digital signal processing (DSP), and implementation of a specific process as hardware to be used with a CPU. The matters disclosed in the present description may be implemented in any form of computer system. The form of computer system does not limit the scope of the present invention. The matters disclosed in the present description may generally be embodied as: (1) a computer program containing an instruction configured to cause, when executed by processing means, an apparatus or a system including the processing means to execute various processes described in the present description; (2) a method for operating the apparatus or the system, the method being performed when the processing means executes the computer program; and (3) the apparatus or the system including the computer program and the processing means configured to execute the computer program.

Note that the pieces of data 132 to 138 are not stored in the auxiliary storage unit 106 in many cases at the time of manufacture, sales, and initial start-up of the system 100. These pieces of data may be transferred from an external apparatus to the system 100 through the network interface 109, for example. In some embodiments, the pieces of data 136 and 138 may be generated and stored when the CPU 102 executes the computer program 120 or other computer programs. In some embodiments of the computer program 120 or the OS 110, the pieces of data 136 and 138 are stored only in the main memory 104 instead of being stored in the auxiliary storage unit 106. In some cases, the data 134 is combined with the data 132, so that the data 134 does not exist as an individual data file. Note that the scope of the present invention is not limited by the existence of the pieces of data 132 to 138.

The following describes the nuclear medicine image data 132 subject to an analysis in an example. The nuclear medicine image data 132 may be image data acquired by PET measurement for diagnosis of bone metastases or other purposes. For example, the nuclear medicine image data 132 may be image data generated on the basis of radiation count values acquired by administering $^{18}$F-NaF as a radiopharmaceutical to a subject, and detecting radiation emitted from within the subject's body with a PET apparatus. In general, each pixel has a value corresponding to a radioactivity count value; that is, each pixel value indicates the intensity of radioactivity. Note that possible nuclear medicine image data subject to an analysis according to the present invention is not limited to PET images acquired using $^{18}$F-NaF. Various nuclear medicine images (PET images, SPECT images, and scintigraphy images) acquired using other radiopharmaceuticals (e.g., Technetium ($^{99m}$Tc) Hydroxymethylenediphosphonate Injection, Technetium ($^{99m}$Tc) Methylenediphosphonate Injection) may be subject to an analysis according to the present invention.

The measurement condition data 134 contains various conditions regarding PET measurement on the basis of which PET images contained in the nuclear medicine image data 132 have been created. The conditions contained in the measurement condition data 134 preferably include at least sex, age, height, and weight of a subject, and also the amount of administered radiopharmaceutical. In some cases, the content of the measurement condition data 134 is contained in the nuclear medicine image data 132, so that the measurement condition data 134 does not exist as an individual data file. In some embodiments, at least one of the conditions contained in the measurement condition data 134 may be input by a user with a keyboard or other devices connected to the peripheral device interface 108. In some embodiments, the measurement condition data 134 may be stored on the main memory 104 instead of the auxiliary storage unit 106.

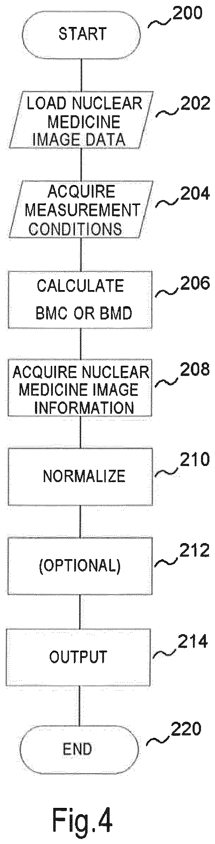

The following describes characteristic processes in a preferred example with reference to FIG. 4. These processes may be performed by the system 100 when the CPU 102 executes the analysis program 120.

Step 200 indicates the start of processing. In step 202, at least part of the nuclear medicine image data 132 is copied to the main memory 104 for preparation of an analysis process to be performed by the analysis program 120.

In step 204, information on measurement conditions on the nuclear medicine image data 132, such as an administered radioactivity dose, is acquired. In one example, this step is performed by loading the measurement condition data 134. In another example, this step is performed by loading information on various conditions contained in the nuclear medicine image data 132. In still another example, various conditions may be input with a keyboard or other devices connected through the peripheral device interface 108. In this case, according to an instruction from the analysis program 120, the CPU 102 may cause, through the display interface 107, a display connected to the display interface 107 to display a message for prompting input of a measurement condition. In some embodiments, the information on measurement conditions acquired in step 204 may include information on a subject (sex, age, weight, height, or other attributes) of the nuclear medicine image data 132.

In step 206, bone mineral content (BMC) or bone mineral density (BMD) is calculated. BMC or BMD can be estimated from sex, age, height, or weight of a subject. A prior research has been conducted specifically on Japanese people using databases (for example, European Journal of Clinical Nutrition (2001) 55, pp. 462-470). According to this document, BMC and BMD can be estimated by the following formulas:

$$\text{Bone mineral content (Male: kg)} = -1.81 - 0.0015 \times \text{Age} + 1.89 \times \text{Height} + 0.017 \times \text{Weight}$$

$$\text{Bone mineral content (Female: kg)} = -1.05 - 0.009 \times \text{Age} + 1.57 \times \text{Height} + 0.017 \times \text{Weight}$$

$$\text{Bone mineral density (Male: g/m}^2) = 0.934 - 0.00081 \times \text{Age} + 0.003 \times \text{Weight}$$

$$\text{Bone mineral density (Female: g/m}^2) = 0.824 - 0.00368 \times \text{Age} + 0.137 \times \text{Height} + 0.0026 \times \text{Weight}$$

In these formulas, the unit of age is year, the unit of weight is kilogram (kg), and the unit of height is meter (m) .

In the present example, the analysis program 120 contains therein these formulas and is configured to control the CPU 102 to substitute, into these formulas, the information on the sex, age, height, and weight of a subject acquired in step 204 and to calculate the bone mineral content BMC and the bone mineral density BMD. In some embodiments, the calculated BMC and/or BMD may be stored, as the data 136 for example, on the auxiliary storage unit 106 or the main memory 104 for example.

Step 208 indicates a phase at which certain information is obtained from the nuclear medicine image data 132. In later steps, this information is normalized with the BMC or the BMD (step 210), and output as a map or a numeric value (step 214). The information acquired in step 208 may vary depending on embodiments. In one embodiment, the information is the pixel value of each pixel. In this case, the data has already been loaded in step 202, and thus step 208 is unnecessary. In some embodiments, the information acquired in step 208 may be the radioactivity concentration of each pixel. In this case, in step 208, each pixel value in the image data 132 may be multiplied by a certain factor, such as the reciprocal of the volume of the pixel, in order to convert the pixel value of each pixel to radioactivity concentration.

In some embodiments, in step 208, correction may be performed to correct the pixel value (count) of each pixel in the nuclear medicine image data 132. This correction may correct the effect of absorption or scatter of radiation within the body, and the attenuation of radioactivity due to the decay of radionuclide. These corrections have generally been performed in nuclear medicine techniques, and thus the detail of the corrections is not described here. In some embodiments, the nuclear medicine image data 132 may have been subjected to such corrections.

In some embodiments, in step 208, calculation may be performed on the radioactivity count value of a region including a plurality of pixels, or the radioactivity concentration of a region including a plurality of pixels (that is, a value obtained by dividing the radioactivity dose in a region including a plurality of pixels by the volume of the region). These values may be subjected to the attenuation correction described above.

In some embodiments, in step 208, a plurality of pieces of information may be calculated. For example, two or more types of information (values) may be calculated out of the radioactivity count value of each pixel, the radioactivity concentration of each pixel, the radioactivity count value of a region including a plurality of pixels, and the radioactivity concentration of a region including a plurality of pixels. These values may be subjected to the attenuation correction described above.

In step 210, the information acquired in step 208 is normalized. Specifically, the information (value) acquired in step 208 is subjected to division, that is, (Administered radioactivity dose ÷ BMC) or (Administered radioactivity dose ÷ BMD), where the administered radioactivity dose is the value acquired in step 204, and the BMC or the BMD is the value acquired in step 206. In some embodiments, the units of the administered radioactivity dose, the BMC, and the BMD may be changed as appropriate.

When the information acquired in step 208 is attenuation-corrected radioactivity concentration for example, then a value obtained by the normalization process in step 210 is the SUVbone defined by formula 2 or 3.

The value normalized in step 210 is output in step 214. In some embodiments, an additional process may be performed before the output. FIG. 4 illustrates such an optional process in step 212. For example, when the information acquired in step 208 is the attenuation-corrected radioactivity concentration of each pixel, which is normalized in step 210 (that is, SUVbones are calculated in step 210), SUVbones in a region of interest including a plurality of pixels may be integrated in step 212.

In step 214, the values calculated in step 210 and/or 212 are output. The output may include visualizing and displaying the values calculated in step 210 and/or 212, and/or displaying the values as numeric values. Alternatively, the output may include storing the values as the data 138 on the auxiliary storage unit 106.

When the values calculated in step 210 are the normalized radioactivity concentration of respective pixels (that is, SUVbones of respective pixels), these values can be displayed as an image in a map view similarly to a tomogram. When the normalized attenuation-corrected radioactivity concentration of a region including a plurality of pixels is calculated in step 210 or 212, it may be displayed as a value. In some embodiments, a map of normalized values of respective pixels and a normalized value of a region of interest may be displayed together. The display may be performed on, for example, a display connected to the display interface 107.

Step 220 indicates the end of the processing.

Embodiments of the present invention have been described with reference to preferred examples; however, the description of these examples are not intended to limit the scope of the present invention, but intended to satisfy the requirements of the Patent Law and to contribute to the understanding of the present invention. The present invention can be embodied in various forms, and embodiments of the present invention include a number of variations other than the embodiments exemplified herein. The present invention is applicable to nuclear medicine images (PET images, SPECT images, and scintigraphy images) acquired using various radiopharmaceuticals other than the PET images acquired using $^{18}$F-NaF described above.

Individual features included in various examples that have been described in the description are not limited to usage with examples in which these features are explicitly explained to be included, but may be used in combination with other examples that have been described herein or various specific embodiments that have not been described. In particular, the processes presented in the flowchart do not necessarily need to be performed in the described order. According to the preference of an executor, the processes may be performed in a changed order or in parallel, as a plurality of blocks integrally implemented or in a manner divided to a plurality of sub-blocks, or in a loop as appropriate. In some embodiments, some of the blocks in the flowchart may be omitted. For example, step 212 may be unnecessary for some embodiments as described above. In other cases, BMC or BMD has already been calculated for some embodiments, and thus recalculation of the BMC or the BMD in step 206 may be unnecessary. The BMC and the BMD may be values separately measured by known methods. Quantification of BMC and BMD may be performed by various known methods (for example, the method described in "SHIKAHOUSHASEN (Dental Radiology), Volume 18 (1978) pp. 278 to 295") other than generally known methods using bone mineral quantification devices. In this case, step 206 can be replaced with a loading process of BMC or BMD. These variations are all included in the scope of the present invention.

The order of the description of the processes defined in the claims does not necessarily specify the mandatory order of the processes. For example, an embodiment specifying a different order of the processes and an embodiment that executes the processes in a loop are also included in the scope of the present invention according to the claims. It should be noted that the applicants claim to possess the right to have a patent granted on all the embodiments not deviating from the spirit of the present invention regardless of whether a patent is claimed in the current set of attached claims.

REFERENCE SIGNS LIST

| | |
|---|---|
| 100 | system |
| 102 | CPU |
| 104 | main memory |
| 106 | auxiliary storage unit |
| 107 | display interface |
| 108 | peripheral device interface |
| 109 | network interface |
| 110 | OS |
| 120 | analysis program |

**Claims**

1. A system for outputting a quantitative value of nuclear medicine image data acquired using a bone disease diagnostic agent, the system comprising:

   first means for loading the nuclear medicine image data;
   second means for loading sex information on a subject of the nuclear medicine image data;
   third means for loading age information on the subject;
   fourth means for loading height information on the subject;
   fifth means for loading weight information on the subject;
   sixth means for loading information on a radioactivity dose administered to the subject;
   seventh means for acquiring certain information from the nuclear medicine image data;
   eighth means for calculating bone mineral content or bone mineral density based on at least one of the sex information acquired by the second means, the age information acquired by the third means, the height information acquired by the fourth means, or the weight information acquired by the fifth means;
   ninth means for normalizing the certain information acquired by the seventh means based on the administered radioactivity dose information acquired by the sixth means, and the bone mineral content or the bone mineral density acquired by the eighth means; and
   tenth means for outputting one or more normalized values acquired by the ninth means,
   the certain information being a value obtained by dividing an amount of attenuation-corrected radioactivity in a region of interest by a volume of the region of interest.

2. The system according to claim 1, wherein the region of interest is a region corresponding to one pixel in the nuclear medicine image data.

3. The system according to claim 1, wherein the tenth means is configured to integrate the normalized values acquired by the ninth means in the region of interest, and to output the integrated value.

4. The system according to any one of claims 1 to 3, wherein the normalized value is SUVbone that is a value defined by the following:

```
SUVbone    =    {Amount    of    attenuation-corrected radioactivity  in  region  of  interest ÷ Volume  of  region  of

interest} / {Administered radioactivity dose ÷ Bone mineral

content},
```

or

$$\text{SUVbone} = \{\text{Amount of attenuation-corrected radioactivity in region of interest} \div \text{Volume of region of interest}\} / \{\text{Administered radioactivity dose} \div \text{Bone mineral density}\}.$$

5. The system according to any one of claims 1 to 4, wherein each of the first to the tenth means is generated when processing means of the system executes a program instruction.

6. A computer program comprising a program instruction that causes, when executed by processing means of a system, the system to perform:

    loading nuclear medicine image data acquired using a bone disease diagnostic agent;
    loading sex information on a subject of the nuclear medicine image data;
    loading age information on the subject;
    loading height information on the subject;
    loading weight information on the subject;
    loading information on a radioactivity dose administered to the subject;
    acquiring certain information from the nuclear medicine image data;
    calculating bone mineral content or bone mineral density based on at least one of the sex information, the age information, the height information, or the weight information;
    normalizing the certain information based on the information on the administered radioactivity dose, and the bone mineral content or the bone mineral density; and
    outputting the normalized information,
    the certain information being a value obtained by dividing an amount of attenuation-corrected radioactivity in a region of interest by a volume of the region of interest.

7. A system for outputting a quantitative value of nuclear medicine image data acquired using a bone disease diagnostic agent, the system comprising:

    first means for loading the nuclear medicine image data;
    second means for loading information on a radioactivity dose administered to a subject of the nuclear medicine image data;
    third means for loading information on bone mineral content or bone mineral density of the subject;
    fourth means for acquiring certain information from the nuclear medicine image data;
    fifth means for normalizing the certain information acquired by the fourth means based on the administered radioactivity dose information acquired by the second means, and the bone mineral content or the bone mineral density acquired by the third means; and
    sixth means for outputting one or more normalized values acquired by the fifth means,
    the certain information being a value obtained by dividing an amount of attenuation-corrected radioactivity in a region of interest by a volume of the region of interest.

8. A computer program comprising a program instruction that causes, when executed by processing means of a system, the system to perform:

    loading nuclear medicine image data acquired using a bone disease diagnostic agent;
    loading information on a radioactivity dose administered to a subject of the nuclear medicine image data;
    loading information on bone mineral content or bone mineral density of the subject;
    acquiring certain information from the nuclear medicine image data;
    normalizing the certain information based on the information on the administered radioactivity dose, and the bone mineral content or the bone mineral density; and
    outputting the normalized information,
    the certain information being a value obtained by dividing an amount of attenuation-corrected radioactivity in a region of interest by a volume of the region of interest.

9. A computer implemented method for outputting a quantitative value of nuclear medicine image data acquired using a bone disease diagnostic agent, comprising:

loading nuclear medicine image data acquired using a bone disease diagnostic agent;
loading sex information on a subject of the nuclear medicine image data;
loading age information on the subject;
loading height information on the subject;
loading weight information on the subject;
loading information on a radioactivity dose administered to the subject;
acquiring certain information from the nuclear medicine image data;
calculating bone mineral content or bone mineral density based on at least one of the sex information, the age information, the height information, or the weight information;
normalizing the certain information based on the information on the administered radioactivity dose, and the bone mineral content or the bone mineral density; and
outputting the normalized information,
the certain information being a value obtained by dividing an amount of attenuation-corrected radioactivity in a region of interest by a volume of the region of interest.

10. The method according to claim 9, wherein the region of interest is a region corresponding to one pixel in the nuclear medicine image data.

11. The method according to claim 9, comprising integrating the normalized values and outputting the integrated value.

12. The method according to claim 9, wherein the normalized value is SUVbone that is a value defined by the following:

```
SUVbone    =    {Amount    of    attenuation-corrected
radioactivity in region of interest ÷ Volume of region of
interest} / {Administered radioactivity dose ÷ Bone mineral
content},
```

or

```
SUVbone    =    {Amount    of    attenuation-corrected
radioactivity in region of interest ÷ Volume of region of
interest} / {Administered radioactivity dose ÷ Bone mineral
density}.
```

**Patentansprüche**

1. System zum Ausgeben eines quantitativen Werts von Nuklearmedizin-Bilddaten, die unter der Verwendung eines Knochenkrankheits-Diagnostikums erhalten wurden, wobei das System umfasst:

ein erstes Mittel zum Laden der Nuklearmedizin-Bilddaten;
ein zweites Mittel zum Laden von Geschlechtsinformationen über einen Patienten der Nuklearmedizin-Bilddaten;
ein drittes Mittel zum Laden von Altersinformationen über den Patienten;
ein viertes Mittel zum Laden von Körpergröße-Informationen über den Patienten;
ein fünftes Mittel zum Laden von Gewichtsinformationen über den Patienten;
ein sechstes Mittel zum Laden von Informationen über eine dem Patienten verabreichte Radioaktivitätsdosis;
ein siebtes Mittel zum Beschaffen bestimmter Informationen aus den Nuklearmedizin-Bilddaten;
ein achtes Mittel zum Berechnen von Knochenmineralgehalt oder Knochenmineraldichte auf Basis mindestens einer aus den von dem zweiten Mittel beschafften Geschlechtsinformationen, den von dem dritten Mittel beschafften Altersinformationen, den von dem vierten Mittel beschafften Körpergröße-Informationen oder den von dem fünften Mittel beschafften Gewichtsinformationen;

ein neuntes Mittel zum Normalisieren der von dem siebten Mittel beschafften bestimmten Informationen auf Basis der von dem sechsten Mittel beschafften Informationen über die verabreichte Radioaktivitätsdosis und des Knochenmineralgehalts oder der Knochenmineraldichte, die von dem achten Mittel beschafft wurden; und ein zehntes Mittel zum Ausgeben eines oder mehrerer von dem neunten Mittel beschafften normalisierten Werte; wobei die bestimmten Informationen ein Wert sind, der durch Teilen einer Menge dämpfungskorrigierter Radioaktivität in einem betreffenden Bereich durch ein Volumen des betreffenden Bereichs erhalten wird.

2.  System gemäß Anspruch 1, wobei der betreffende Bereich ein Bereich ist, der einem Pixel in den Nuklearmedizin-Bilddaten entspricht.

3.  System gemäß Anspruch 1, wobei das zehnte Mittel dazu konfiguriert ist, die von dem neunten Mittel in dem betreffenden Bereich beschafften normalisierten Werte zu integrieren und den integrierten Wert auszugeben.

4.  System gemäß einem der Ansprüche 1 bis 3, wobei der normalisierte Wert SUVbone ist, der ein Wert ist, der durch das Folgende definiert ist:

$$SUVbone = \{\text{Menge an dämpfungskorrigierter Radioaktivität in betreffendem Bereich} \div \text{Volumen des betreffenden Bereichs}\} / \{\text{verabreichte Radioaktivitätsdosis} \div \text{Knochenmineralgehalt}\},$$

oder

$$SUVbone = \{\text{Menge an dämpfungskorrigierter Radioaktivität in betreffendem Bereich} \div \text{Volumen des betreffenden Bereichs}\} / \{\text{verabreichte Radioaktivitätsdosis} \div \text{Knochenmineraldichte}\}.$$

5.  System gemäß einem der Ansprüche 1 bis 4, wobei jedes aus dem ersten bis zum zehnten Mittel erzeugt wird, wenn ein Verarbeitungsmittel des Systems einen Programmbefehl ausführt.

6.  Computerprogramm, umfassend einen Programmbefehl, der bei einer Ausführung von einem Verarbeitungsmittel eines Systems verursacht, dass das System ausführt:

Laden von Nuklearmedizin-Bilddaten, die unter der Verwendung eines Knochenkrankheits-Diagnostikums beschafft wurden;
Laden von Geschlechtsinformationen über einen Patienten der Nuklearmedizin-Bilddaten;
Laden von Altersinformationen über den Patienten;
Laden von Körpergröße-Informationen über den Patienten;
Laden von Gewichtsinformationen über den Patienten;

Laden von Informationen über eine dem Patienten verabreichte Radioaktivitätsdosis;
Beschaffen bestimmter Informationen aus den Nuklearmedizin-Bilddaten;
Berechnen von Knochenmineralgehalt oder Knochenmineraldichte auf Basis mindestens einer aus den Geschlechtsinformationen, den Altersinformationen, den Körpergröße-Informationen oder den Gewichtsinformationen;
Normalisieren der bestimmten Informationen auf Basis der Informationen über die verabreichte Radioaktivitätsdosis und des Knochenmineralgehalts oder der Knochenmineraldichte; und
Ausgeben der normalisierten Informationen;
wobei die bestimmten Informationen ein Wert sind, der durch Teilen einer Menge dämpfungskorrigierter Radioaktivität in einem betreffenden Bereich durch ein Volumen des betreffenden Bereichs erhalten wird.

7.  System zum Ausgeben eines quantitativen Werts von Nuklearmedizin-Bilddaten, die unter der Verwendung eines Knochenkrankheits-Diagnostikums erhalten wurden, wobei das System umfasst:

ein erstes Mittel zum Laden der Nuklearmedizin-Bilddaten;

ein zweites Mittel zum Laden von Informationen über eine Radioaktivitätsdosis, die einem Patienten der Nuklearmedizin-Bilddaten verabreicht wurde;

ein drittes Mittel zum Laden von Informationen über Knochenmineralgehalt oder Knochenmineraldichte des Patienten;

ein viertes Mittel zum Beschaffen bestimmter Informationen aus den Nuklearmedizin-Bilddaten;

ein fünftes Mittel zum Normalisieren der von dem vierten Mittel beschafften bestimmten Informationen auf Basis der von dem zweiten Mittel beschafften Informationen über die verabreichte Radioaktivitätsdosis und des Knochenmineralgehalts oder der Knochenmineraldichte, die von dem dritten Mittel beschafft wurden; und

ein sechstes Mittel zum Ausgeben eines oder mehrerer der von dem fünften Mittel beschafften normalisierten Werte,

wobei die bestimmten Informationen ein Wert sind, der durch Teilen einer Menge dämpfungskorrigierter Radioaktivität in einem betreffenden Bereich durch ein Volumen des betreffenden Bereichs erhalten wird.

8. Computerprogramm, umfassend einen Programmbefehl, der bei einer Ausführung von einem Verarbeitungsmittel eines Systems verursacht, dass das System ausführt:

Laden von Nuklearmedizin-Bilddaten, die unter der Verwendung eines Knochenkrankheit-Diagnostikums beschafft wurden;

Laden von Informationen über eine Radioaktivitätsdosis, die einem Patienten der Nuklearmedizin-Bilddaten verabreicht wurde;

Laden von Informationen über Knochenmineralgehalt oder Knochenmineraldichte des Patienten;

Beschaffen bestimmter Informationen aus den Nuklearmedizin-Bilddaten;

Normalisieren der bestimmten Informationen auf Basis der Informationen über die verabreichte Radioaktivitätsdosis und des Knochenmineralgehalts oder der Knochenmineraldichte; und

Ausgeben der normalisierten Informationen,

wobei die bestimmten Informationen ein Wert sind, der durch Teilen einer Menge dämpfungskorrigierter Radioaktivität in einem betreffenden Bereich durch ein Volumen des betreffenden Bereichs erhalten wird.

9. Computerimplementiertes Verfahren zum Ausgeben eines quantitativen Werts von Nuklearmedizin-Bilddaten, die unter der Verwendung eines Knochenkrankheits-Diagnostikums erhalten wurden, umfassend:

Laden von Nuklearmedizin-Bilddaten, die unter der Verwendung eines Knochenkrankheits-Diagnostikums beschafft wurden;

Laden von Geschlechtsinformationen über einen Patienten der Nuklearmedizin-Bilddaten;

Laden von Altersinformationen über den Patienten;

Laden von Körpergröße-Informationen über den Patienten;

Laden von Gewichtsinformationen über den Patienten;

Laden von Informationen über eine dem Patienten verabreichte Radioaktivitätsdosis;

Beschaffen bestimmter Informationen aus den Nuklearmedizin-Bilddaten;

Berechnen von Knochenmineralgehalt oder Knochenmineraldichte auf Basis mindestens eines aus den Geschlechtsinformationen, den Altersinformationen, den Körpergröße-Informationen oder den Gewichtsinformationen;

Normalisieren der bestimmten Informationen auf Basis der Informationen über die verabreichte Radioaktivitätsdosis und des Knochenmineralgehalts oder der Knochenmineraldichte; und

Ausgeben der normalisierten Informationen;

wobei die bestimmten Informationen ein Wert sind, der durch Teilen einer Menge dämpfungskorrigierter Radioaktivität in einem betreffenden Bereich durch ein Volumen des betreffenden Bereichs erhalten wird.

10. Verfahren gemäß Anspruch 9, wobei der betreffende Bereich ein Bereich ist, der einem Pixel in den Nuklearmedizin-Bilddaten entspricht.

11. Verfahren gemäß Anspruch 9, umfassend Integrieren der normalisierten Werte und Ausgeben des integrierten Werts.

12. Verfahren gemäß Anspruch 9, wobei der normalisierte Wert SUVbone ist, der ein Wert ist, der durch das Folgende definiert ist:

$$\text{SUVbone} = \{\text{Menge an dämpfungskorrigierter Radioaktivität in betreffendem Bereich} \div \text{Volumen des betreffenden Bereichs}\} / \{\text{verabreichte Radioaktivitätsdosis} \div \text{Knochenmineralgehalt}\},$$

oder

$$\text{SUVbone} = \{\text{Menge an dämpfungskorrigierter Radioaktivität in betreffendem Bereich} \div \text{Volumen des betreffenden Bereichs}\} / \{\text{verabreichte Radioaktivitätsdosis} \div \text{Knochenmineraldichte}\}.$$

**Revendications**

1. Système pour fournir en sortie une valeur quantitative de données d'images de médecine nucléaire acquises à l'aide d'un agent de diagnostic de maladie osseuse, le système comprenant :

   des premiers moyens pour charger les données d'images de médecine nucléaire ;
   des deuxièmes moyens pour charger une indication du sexe d'un sujet des données d'images de médecine nucléaire ;
   des troisièmes moyens pour charger une indication de l'âge du sujet ;
   des quatrièmes moyens pour charger une indication de la taille du sujet ;
   des cinquièmes moyens pour charger une indication du poids du sujet ;
   des sixièmes moyens pour charger une indication d'une dose de radioactivité administrée au sujet ;
   des septièmes moyens pour acquérir certaines indications à partir des données d'images de médecine nucléaire ;
   des huitièmes moyens pour calculer un contenu minéral osseux ou une densité minérale osseuse sur la base d'au moins une parmi l'indication du sexe acquise par les deuxièmes moyens, l'indication de l'âge acquise par les troisièmes moyens, l'indication de la taille acquise par les quatrièmes moyens ou l'indication du poids acquise par les cinquièmes moyens ;
   des neuvièmes moyens pour normaliser les certaines indications acquises par les septièmes moyens sur la base de l'indication de la dose de radioactivité administrée acquise par les sixièmes moyens, et du contenu minéral osseux ou de la densité minérale osseuse acquis par les huitièmes moyens ; et
   des dixièmes moyens pour fournir en sortie une ou plusieurs valeurs normalisées acquises par les neuvièmes moyens,
   les certaines indications étant une valeur obtenue en divisant une quantité de radioactivité corrigée en atténuation dans une région d'intérêt par un volume de la région d'intérêt.

2. Système selon la revendication 1, dans lequel la région d'intérêt est une région correspondant à un pixel dans les données d'images de médecine nucléaire.

3. Système selon la revendication 1, dans lequel les dixièmes moyens sont conçus pour intégrer les valeurs normalisées acquises par les neuvièmes moyens dans la région d'intérêt, et pour fournir en sortie la valeur intégrée.

4. Système selon l'une quelconque des revendications 1 à 3, dans lequel la valeur normalisée est SUVbone qui est une valeur définie par les expressions suivantes :

$$\text{SUVbone} = \{\text{Quantité de radioactivité corrigée en atténuation dans la région d'intérêt} \div \text{Volume de la région d'intérêt}\} / \{\text{Dose de radioactivité administrée} \div \text{Contenu minéral osseux}\},$$

ou

$$SUVbone = \{\text{Quantité de radioactivité corrigée en atténuation dans la région d'intérêt} \div \text{Volume de la région d'intérêt}\} / \{\text{Dose de radioactivité administrée} \div \text{Densité minérale osseuse}\}.$$

5. Système selon l'une quelconque des revendications 1 à 4, dans lequel chacun des premiers aux dixièmes moyens est généré lorsque des moyens de traitement du système exécutent une instruction de programme.

6. Programme informatique comprenant une instruction de programme qui amène, lorsqu'elle est exécutée par des moyens de traitement d'un système, le système à effectuer :

le chargement de données d'images de médecine nucléaire acquises à l'aide d'un agent de diagnostic de maladie osseuse ;
le chargement d'une indication du sexe d'un sujet des données d'images de médecine nucléaire ;
le chargement d'une indication de l'âge du sujet ;
le chargement d'une indication de la taille du sujet ;
le chargement d'une indication du poids du sujet ;
le chargement d'une indication d'une dose de radioactivité administrée au sujet ;
l'acquisition de certaines indications à partir des données d'images de médecine nucléaire ;
le calcul d'un contenu minéral osseux ou d'une densité minérale osseuse sur la base d'au moins une parmi l'indication du sexe, l'indication de l'âge, l'indication de la taille ou l'indication du poids ;
la normalisation des certaines indications sur la base de l'indication de la dose de radioactivité administrée, et du contenu minéral osseux ou de la densité minérale osseuse ; et
la fourniture en sortie des indications normalisées,
les certaines indications étant une valeur obtenue en divisant une quantité de radioactivité corrigée en atténuation dans une région d'intérêt par un volume de la région d'intérêt.

7. Système pour fournir en sortie une valeur quantitative de données d'images de médecine nucléaire acquises à l'aide d'un agent de diagnostic de maladie osseuse, le système comprenant :

des premiers moyens pour charger les données d'images de médecine nucléaire ;
des deuxièmes moyens pour charger une indication d'une dose de radioactivité administrée à un sujet des données d'images de médecine nucléaire ;
des troisièmes moyens pour charger une indication du contenu minéral osseux ou de la densité minérale osseuse du sujet ;
des quatrièmes moyens pour acquérir certaines indications à partir des données d'images de médecine nucléaire ;
des cinquièmes moyens pour normaliser les certaines indications acquises par les quatrièmes moyens sur la base de l'indication de la dose de radioactivité administrée acquise par les deuxièmes moyens, et du contenu minéral osseux ou de la densité minérale osseuse acquis par les troisièmes moyens ; et
des sixièmes moyens pour fournir en sortie une ou plusieurs valeurs normalisées acquises par les cinquièmes moyens,
les certaines indications étant une valeur obtenue en divisant une quantité de radioactivité corrigée en atténuation dans une région d'intérêt par un volume de la région d'intérêt.

8. Programme informatique comprenant une instruction de programme qui amène, lorsqu'elle est exécutée par des moyens de traitement d'un système, le système à effectuer :

le chargement de données d'images de médecine nucléaire acquises à l'aide d'un agent de diagnostic de maladie osseuse ;
le chargement d'une indication d'une dose de radioactivité administrée à un sujet des données d'images de médecine nucléaire ;
le chargement d'une indication du contenu minéral osseux ou de la densité minérale osseuse du sujet ;
l'acquisition de certaines indications à partir des données d'images de médecine nucléaire ;
la normalisation des certaines indications sur la base de l'indication de la dose de radioactivité administrée, et du contenu minéral osseux ou de la densité minérale osseuse ; et
la fourniture en sortie de l'indication normalisée,

les certaines indications étant une valeur obtenue en divisant une quantité de radioactivité corrigée en atténuation dans une région d'intérêt par un volume de la région d'intérêt.

9. Procédé mis en oeuvre par ordinateur pour fournir en sortie une valeur quantitative de données d'images de médecine nucléaire acquises à l'aide d'un agent de diagnostic de maladie osseuse, comprenant :

le chargement de données d'images de médecine nucléaire acquises à l'aide d'un agent de diagnostic de maladie osseuse ;
le chargement d'une indication du sexe d'un sujet des données d'images de médecine nucléaire ;
le chargement d'une indication de l'âge du sujet ;
le chargement d'une indication de la taille du sujet ;
le chargement d'une indication du poids du sujet ;
le chargement d'une indication d'une dose de radioactivité administrée au sujet ;
l'acquisition de certaines indications à partir des données d'images de médecine nucléaire ;
le calcul d'un contenu minéral osseux ou d'une densité minérale osseuse sur la base d'au moins une parmi l'indication du sexe, l'indication de l'âge, l'indication de la taille ou l'indication du poids ;
la normalisation des certaines indications sur la base de l'indication de la dose de radioactivité administrée, et du contenu minéral osseux ou de la densité minérale osseuse; et
la fourniture en sortie de l'indication normalisée,
les certaines indications étant une valeur obtenue en divisant une quantité de radioactivité corrigée en atténuation dans une région d'intérêt par un volume de la région d'intérêt.

10. Procédé selon la revendication 9, dans lequel la région d'intérêt est une région correspondant à un pixel dans les données d'images de médecine nucléaire.

11. Procédé selon la revendication 9, comprenant l'intégration des valeurs normalisées et la fourniture en sortie de la valeur intégrée.

12. Procédé selon la revendication 9, dans lequel la valeur normalisée est SUVbone qui est une valeur définie par les expressions suivantes :

$$\text{SUVbone} = \{\text{Quantité de radioactivité corrigée en atténuation dans la région d'intérêt} \div \text{Volume de la région d'intérêt}\} / \{\text{Dose de radioactivité administrée} \div \text{Contenu minéral osseux}),$$

ou

$$\text{SUVbone} = \{\text{Quantité de radioactivité corrigée en atténuation dans la région d'intérêt} \div \text{Volume de la région d'intérêt}\} / \{\text{Dose de radioactivité administrée} \div \text{Densité minérale osseuse}\}.$$

Fig.1

EP 3 168 650 B1

$$y = 0.0557x + 1.813$$
$$R^2 = 0.3584$$

$$y = 0.0009x + 0.1371$$
$$R^2 = 0.0854$$

Fig.2A

AGE

Fig.2B

100

107 — DISPLAY

102

104 — RAM

108 — PERIPHERAL DEVICE

CPU

109 — NETWORK

106

OS ~110

ORIGINAL IMAGE DATA ~132

ANALYSIS PROGRAM ~120

MEASUREMENT CONDITION DATA ~134

BMC, BMD ~136

PROCESSING RESULT DATA ~138

Fig.3

```
          ┌───────────────┐
          │     START     │ ∿ 200
          └───────┬───────┘
                  │
          ┌───────────────┐
         / LOAD NUCLEAR  / ∿ 202
        /   MEDICINE    /
       /  IMAGE DATA   /
          └───────┬───────┘
                  │
          ┌───────────────┐
         /   ACQUIRE     / ∿ 204
        /  MEASUREMENT  /
       /  CONDITIONS   /
          └───────┬───────┘
                  │
          ┌───────────────┐
          │   CALCULATE   │ ∿ 206
          │  BMC OR BMD   │
          └───────┬───────┘
                  │
          ┌───────────────┐
          │ACQUIRE NUCLEAR│ ∿ 208
          │MEDICINE IMAGE │
          │  INFORMATION  │
          └───────┬───────┘
                  │
          ┌───────────────┐
          │   NORMALIZE   │ ∿ 210
          └───────┬───────┘
                  │
          ┌───────────────┐
          │  (OPTIONAL)   │ ∿ 212
          └───────┬───────┘
                  │
          ┌───────────────┐
          │    OUTPUT     │ ∿ 214
          └───────┬───────┘
                  │
          ┌───────────────┐
          │     END       │ ∿ 220
          └───────────────┘
```

Fig.4

# EP 3 168 650 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YOSHIFUMI SUGAWARA ; KENNETH R. ZASAD-NY et al.** Reevaluation of the Standardized Uptake Value for FDG: Variations with Body Weight and Methods for Correction. *Radiology,* November 1999, vol. 213, 521-525

- *European Journal of Clinical Nutrition,* 2001, vol. 55, 462-470
- *SHIKAHOUSHASEN (Dental Radiology),* 1978, vol. 18, 278-295